(19) Europäisches Patentamt
European Patent Office
Office européen des brevets

(11) EP 0 744 172 B1

(12) **EUROPÄISCHE PATENTSCHRIFT**

(45) Veröffentlichungstag und Bekanntmachung des Hinweises auf die Patenterteilung:
**02.04.2003 Patentblatt 2003/14**

(51) Int Cl.[7]: **A61K 6/02**, A61K 6/083

(21) Anmeldenummer: **96250116.9**

(22) Anmeldetag: **24.05.1996**

(54) **Photochromer Dentalwerkstoff**

Photochromic dental material

Composition dentaire photochromique

(84) Benannte Vertragsstaaten:
**AT CH DE FR GB IT LI SE**

(30) Priorität: **26.05.1995 DE 19520016**

(43) Veröffentlichungstag der Anmeldung:
**27.11.1996 Patentblatt 1996/48**

(73) Patentinhaber: **Ivoclar Vivadent AG**
**9494 Schaan (LI)**

(72) Erfinder:
- **Salz, Ulrich, Dr.**
  **88131 Lindau (DE)**
- **Burtscher, Peter,Dr.**
  **6830 Rankweil (AT)**
- **Rheinberger, Volker, Dr.**
  **9490 Vaduz (LI)**
- **Dürr, Heinz, Prof. Dr.**
  **66121 Saarbrücken (DE)**

(74) Vertreter: **UEXKÜLL & STOLBERG**
**Patentanwälte**
**Beselerstrasse 4**
**22607 Hamburg (DE)**

(56) Entgegenhaltungen:
**EP-A- 0 486 775**
**WO-A-93/17632**
**DE-A- 3 036 103**
**DE-A- 3 717 762**
**US-A- 4 600 389**

- **CHEMICAL ABSTRACTS, vol. 113, no. 20, 12. November 1990 (1990-11-12) Columbus, Ohio, US; abstract no. 176669, KOKUBO, TADASHI: "Formation of crystalline fine particles from amorphous solid phase and its application" XP002112725 & FUNSAI (1990), (34), 117-22 ,1990,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Dentalwerkstoffe, die ein photochromes Material wie beispielsweise einen photochromen Farbstoff, ein photochromes Glas, eine photochrome Keramik und/oder eine photochrome Glaskeramik enthalten und welche sich nach Bestrahlung mit Licht visuell vom natürlichen Zahnmaterial unterscheiden lassen.

In der restaurativen Zahnheilkunde werden aus ästhetischen Gründen in zunehmendem Maße zahnfarbene Restaurationswerkstoffe eingesetzt. Diese Werkstoffe haben den Nachteil, daß sie visuell nur schwer von der natürlichen Zahnsubstanz zu unterscheiden sind, so daß das Entfernen von überschüssigem Material sowie die Nachbearbeitung und Anpassung beispielsweise von Füllungen erschwert wird. Das hat zur Folge, daß häufig unnötigerweise gesunde Zahnsubstanz entfernt oder andererseits überschüssiger Dentalwerkstoff übersehen wird, welcher dann als Retentionsnische die Bildung von Plaque begünstigen und zu Parodontalproblemen führen kann. Auch bei der Entfernung zahnfarbener Füllungen wird häufig wegen der schlechten Sichtbarkeit des Übergangs zwischen Füllung und Zahnsubstanz entweder zu viel gesunde Zahnsubstanz entfernt oder aber Reste der Füllung werden übersehen.

Ähnliche Probleme ergeben sich bei der Verwendung von zahnfarbenen Befestigungsmaterialien zur Zementierung von zahnfarbenen Restaurationen.

In der US-PS 5 162 130 werden Dentalwerkstoffe offenbart, die ein photosensitives Material enthalten. Diese Dentalwerkstoffe erlauben die Herstellung von Dentalrestaurationen, die sich durch Bestrahlen mit UV-Licht und anschließendes Erhitzen farblich an ihre Umgebung angleichen lassen. Da die Restaurationen durch die Bestrahlung und das Erhitzen permanent verfärbt werden, eignen sich die photosensitiven Materialien nicht zur vorübergehenden Sichtbarmachung von farblosen oder zahnfarbenen Dentalwerkstoffen.

Aus der US-PS 4 600 389 sind Dentalwerkstoffe bekannt, die fluoreszierende Lanthanidenverbindungen enthalten, welche bei Bestrahlung mit einer Quecksilberdampflampe eine rötliche bzw. grünliche Fluoreszenz zeigen und so eine Unterscheidung von Dentalwerkstoff und Zahnsubstanz erlauben.

Die GB 2 190 917 offenbart ein farbiges bzw. fluoreszierendes Überzugsmaterial für Zähne, das einen entfernbaren Schutzfilm bildet.

Die DE 39 39 998 A1 betrifft ein Verfahren zur optischen Unterscheidung zwischen Dentalwerkstoff und natürlichem Zahnmaterial, das auf der Verwendung eines fluoreszierenden Stoffes und spezieller Lichtfilter basiert.

Die GB 2 230 271 A offenbart einen Dentalwerkstoff, der einen Farbstoff enthält, der sich durch sichtbares Licht zur Fluoreszenz anregen läßt.

Fluoreszierende Dentalwerkstoffe haben den Nachteil, daß die Fluoreszenz nur bei gleichzeitiger Bestrahlung mit einer geeigneten Lichtquelle auftritt, so daß neben den üblichen Arbeitsgeräten des Zahnarztes zusätzlich ein Lichtleiter in der Mundhöhle Platz finden muß, wodurch die Arbeit des Arztes in der engen Mundhöhle erschwert wird. Zudem ist häufig der Einsatz spezieller Lampen erforderlich. Darüber hinaus zeigt die natürliche Zahnsubstanz eine starke Eigenfluoreszenz, so daß relativ hohe Konzentrationen des Fluoreszenzfarbstoffes in dem Dentalwerkstoff erforderlich sind, um eine gute Unterscheidbarkeit von Dentalwerkstoff und Zahnsubstanz sicherzustellen. Dies hat vor allem bei Fluoreszenzfarbstoffen mit einem Absorptionsmaximum oberhalb von 400 nm eine deutlich sichtbare Verfärbung des Dentalwerkstoffs durch den Fluoreszenzfarbstoff zur Folge.

T. Kokubo, Funsai (1990), Nr. 34, 117-122, beschreibt die Bildung von kristallinen Partikeln aus amorphen Festkörpern, wie beispielsweise mit AgCl dotierten photochromen Gläsern oder Glaskeramiken für dentale Anwendungen.

Aufgabe der vorliegenden Erfindung ist die Schaffung eines Dentalwerkstoffs, dessen Farbe sich durch die kurzzeitige Bestrahlung mit einer geeigneten Lichtquelle so ändern läßt, daß eine problemlose visuelle Unterscheidung des Dentalwerkstoffs von der natürlichen Zahnsubstanz gewährleistet ist, und welcher nach einem zur Entfernung überschüssigen Dentalwerkstoffs oder zur Bearbeitung des Dentalwerkstoffs ausreichenden Zeitraum wieder seine ursprüngliche Farbe annimmt.

Diese Aufgabe wird durch Dentalwerkstoffe gelöst, welche zusätzlich ein photochromes Material, wie beispielsweise einen photochromen Farbstoff, ein photochromes Glas, eine photochrome Keramik und/oder eine photochrome Glaskeramik enthalten.

Unter Photochromie wird ein reversibler Übergang einer chemischen Substanz zwischen zwei Zuständen mit unterschiedlichen Absorptionsspektren verstanden, wobei der Übergang zumindest in einer Richtung durch elektromagnetische Strahlung verursacht wird.

$$A\ (\lambda 1) \overset{h\nu}{\rightleftharpoons} B\ (\lambda 2)$$

Bei der Bestrahlung mit Licht im Wellenlägenbereich des Absorptionsmaximums λ1 des Ausgangszustands

A geht die Substanz in die energiereichere intensiv gefärbte Form B über. Die Rückreaktion von B zu A verläuft meist spontan und im Vergleich zur Hinreaktion mit geringerer Geschwindigkeit.

Bevorzugte photochrome Materialien sind photochrome Farbstoffe und photochrome Gläser sowie photochrome Keramiken oder Glaskeramiken.

Geeignete photochrome Farbstoffe werden beispielsweise in *Photochromism - Molecules and Systems* (Dürr, H.; Bouas-Laurent, H., Herausgeber, Elsevier, 1990) beschrieben. Bevorzugte photochrome Farbstoffsysteme basieren auf der cis/trans-Isomerie von Azobenzolverbindungen oder Stilbenen, der Interconversion oder elektrocyclischen Ringschluß-/Ringöffnungsreaktion von Spiropyran-Systemen oder Spirooxazinen zu Merocyaninen, oder der 1,5-Elektrocyclisierung von Pentadienyl-Anionen.

Eine bevorzugte Gruppe photochromer Farbstoffe sind Spiro[1,8aindolizin]-Derivate, insbesondere Spiro [1,8a-dihydroindolizin]-und Spiro[1,8a-tetrahydroindolizin]-Derivate. Geeignete Derivate und Verfahren zu deren Herstellung werden beispielsweise in der DE 29 06 193 C2 und der DE 32 20 257 C2 offenbart.

Besonders bevorzugt sind Systeme, die auf einer 1,5-Electrocyclisierung basieren, wie sie von H. Dürr in *Angew. Chem.* **101** (1989), Seiten 427 bis 445 in Kapitel 3 beschrieben werden.

Ganz besonders bevorzugt sind Spiro[fluoren-9,1'[1,8a]dihydroindolizin]-Derivate, insbesondere Derivate gemäß der Formel

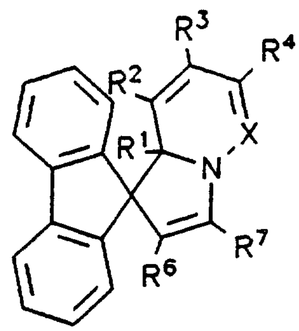

I

in der

X C-$R^5$ oder N;
$R^1$ H oder $CH_3$;
$R^2$ H, $CH_2$=C($CH_3$)-COO- oder zusammen mit $R^3$ einen ankondensierten Benzolring (-($CH_2$=$CH_2$-)$_2$);
$R^3$ H, $CH_3$, $COOCH_3$, CN, $CH_2$=C($CH_3$)-COO- oder zusammen mit $R^2$ einen ankondensierten Benzolring (-($CH_2$=$CH_2$-)$_2$);
$R^4$ H oder $CH_3$;
$R^5$ H oder $CH_2$=C($CH_3$)-COO-;
$R^6$ $COOCH_3$ oder $COCH_3$ und
$R^7$ $COOCH_3$ oder $COCH_3$

bedeuten.

Bevorzugte Substituenten sind X = N; $R^1$ = H; $R^2$ = = H; $R^3$ = H; $R^4$ = H oder $CH_3$; $R^6$ = $COOCH_3$; $R^7$ = $COOCH_3$.

Ganz besonders bevorzugte Systeme sind X = N, $R^1$ = $R^2$ = $R^3$ = H; $R^4$ = $CH_3$; $R^6$ = $R^7$ = $COOCH_3$ (1'-H-2',3'-Dicarbomethoxy-5'-methylspiro[fluoren-9,1'-pyrrolo-[1,2-B]-pyridazin]); X = N, $R^1$ = $R^2$ = $R^3$ = $R^4$ = H; $R^6$ = $R^7$ = $COOCH_3$; X = N, $R^1$ = $CH_3$, $R^2$ = $R^3$ = H, $R^4$ = $CH_3$; $R^6$ = $R^7$ = $COOCH_3$; und X = N, $R^1$ = $R^2$ = $R^3$ = $R^4$ = H; $R^6$ = $COCH_3$, $R^7$ = $COOCH_3$.

Die Verfärbung der erfindungsgemäß bevorzugten Farbstoffe ist auf das bei der Bestrahlung entstehende Betain II (Zustand B) zurückzuführen:

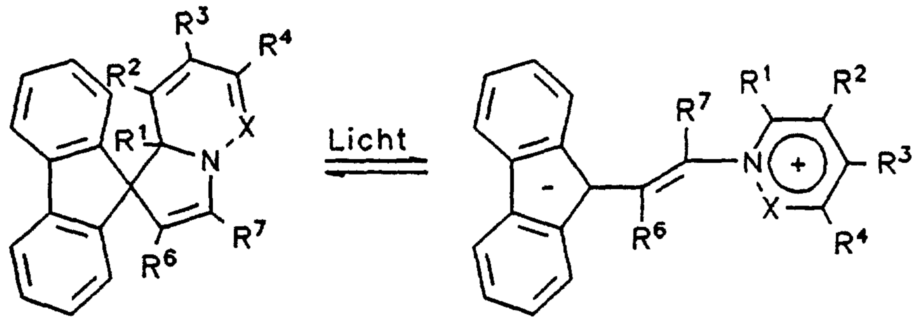


I II

Photochrome Farbstoffe werden vorzugsweise in einer Menge von 0,0001 bis 0,1 Gew.-%, besonders bevorzugt 0,002 bis 0,01 Gew.-% bezogen auf die Gesamtmasse des Dentalwerkstoffs eingesetzt. Erfindungsgemäß sind solche Farbstoffe bevorzugt, deren Absorptionsmaximum $\lambda 1$ des Zustands A im Wellenlängenbereich handelsüblicher Polymerisationslampen für Dentalkomposite, vorzugsweise im Bereich von 400 bis 500 nm, liegt. Das Absorptionsmaximum $\lambda 2$ des aktivierten Zustandes B liegt im sichtbaren Wellenlängenbereich, so daß der Dentalwerkstoff nach der Bestrahlung farbig erscheint und visuell leicht von der natürlichen Zahnsubstanz unterschieden werden kann.

Zur Herstellung von photohärtbaren Dentalwerkstoffen sind weiter solche Farbstoffe bevorzugt, die zur Erzeugung des farbigen Zustands B eine Bestrahlungszeit erfordern, die kleiner als die zur Auslösung der Polymerisation erforderliche Zeit ist, so daß der Dentalwerkstoff durch eine kurze Bestrahlung gefärbt und überschüssiges Material im noch ungehärteten aber gefärbten Zustand entfernt werden kann. Besonders bevorzugt sind daher photochrome Materialien, die zur Erzeugung des gefärbten Zustandes B eine Bestrahlungszeit von maximal 1 bis 3 Sekunden erfordern.

Zur Anhärtung wird der Dentalwerkstoff ein weiteres Mal z.B. für 10 Sekunden bestrahlt, wobei der ausgehärtete Werkstoff gefärbt und damit leicht erkennbar bleibt. Dann ist der Dentalwerkstoff gefärbt; zur Aushärtung wird er wieder entfärbt. Es ist jedoch prinzipiell auch möglich, den Dentalwerkstoff durch eine einmalige längere Bestrahlung sofort zu härten.

Selbsthärtende Dentalwerkstoffe benötigen zur Härtung gewöhnlich mehrere Minuten, üblicherweise etwa 2 bis 4 Minuten. Auch hier sind photochrome Materialien bevorzugt, die zur Erzeugung des gefärbten Zustandes eine Bestrahlungszeit von maximal 1 bis 3 Sekunden erfordern, so daß überschüssiger Werkstoff im ungehärteten Zustand entfernt werden kann.

Die erfindungsgemäßen photochromen Farbstoffe eignen sich sowohl zur Herstellung von reversibel als auch von irreversibel entfärbbaren Dentalwerkstoffen.

Irreversible entfärbbare Dentalwerkstoffe eignen sich besonders zur Zementierung von zahnfarbenen Keramikrestaurationen (zum Beispiel Inlays, Onlays und Kronen). Hier ist es häufig erwünscht, daß sich der zur Zementierung verwendete photochrome Zement nur bei der Zementierung verfärbt, nach der Zementierung aber dauerhaft farblos bzw. zahnfarben ist, um vor allem im Frontzahnbereich eine Verfärbung des Zements beispielsweise durch Sonneneinstrahlung zu vermeiden.

Zur Herstellung von irreversibel entfärbbaren Dentalwerkstoffen eignen sich besonders die oben aufgeführten Spiro[1,8a-indolizin]-Derivate, da diese im Verlauf der radikalischen Polymerisation des Dentalwerkstoffs häufig zerstört werden, was eine nicht reversible Entfärbung zur Folge hat. Diese Eigenschaft erlaubt zudem eine gezielte Entfärbung des Dentalwerkstoffs nach Fertigstellung der Dentalrestauration.

Da bei irreversibel entfärbbaren Dentalwerkstoffen die Härtung des Werkstoffs aufgrund der Zerstörung des photochromen Farbstoffs mit einer teilweisen Entfärbung des Werkstoffs verbunden ist, empfiehlt sich in diesen Fällen eine Härtung in zwei Stufen.

Nach der ersten kurzzeitigen Bestrahlung des Dentalwerkstoffs zur Erzeugung des gefärbten Zustands und ggf. nach dem Entfernen bzw. Bearbeiten des ungehärteten Dentalwerkstoffs wird dieser zur oberflächlichen Härtung ein zweites Mal bestrahlt. Hierzu ist meist eine Bestrahlungszeit von etwa 10 Sekunden ausreichend. Anschließend können ggf. die ausgehärteten aber noch gefärbten Überschüsse des Dentalwerkstoffs enfernt werden. Danach wird der Dentalwerkstoff durch eine längere Bestrahlung von vorzugsweise 40 bis 60 Sekunden vollständig ausgehärtet, wobei eine weitgehende Entfärbung stattfindet.

Die vollständige Entfärbung des Werkstoffs erfolgt durch die folgende Rückreaktion nicht zerstörten Fabstoffmoleküle zum Ausgangszustand A und ggf. durch das Entfernen der Oberflächenschicht des Werkstoffs. In der Oberflächenschicht wird die radikalische Polymerisation häufig durch eindiffundierenden Sauerstoff inhibiert, so daß die Polymerisation und damit die Zerstörung des photochromen Farbstoffs in dieser Schicht unvollständig ist. In der Praxis wird diese Schicht, welche gewöhnlich eine Stärke von etwa 100 µm aufweist, beim Polieren der Füllungen und Zementränder entfernt.

Die Zeit bis zur vollständigen Entfärbung des Werkstoffs hängt von der Art und der Menge des verwendeten Farbstoffs ab. Im Fall der erfindungsgemäß bevorzugten Farbstoffe wird der Werkstoff bei einer Farbstoffmenge von etwa 0,002 Gew.-% bereits im Verlauf der Bestrahlung von 40 bis 60 Sekunden vollständig entfärbt, während bei einer Farbstoffmenge von etwa 0,01 Gew.-% die vollständige Entfärbung nach einer Bestrahlung von 40 bis 60 Sekunden innerhalb von 24 Stunden unter Lichtausschluß stattfindet.

Dauerhaft reversible photochrome Dentalwerkstoffe werden bei der Kombination organischer photochromer Farbstoffe mit heißhärtenden Kompositen erhalten, wenn der Dentalwerkstoff im ungefärbten Zustand gehärtet wird. Im Fall der erfindungsgemäß bevorzugten photochromen Farbstoffe, deren Photochromie auf einer 1,5-Elektrocyclisierung beruht, ist dies vermutlich darauf zurückzuführen, daß die Farbstoffe nur in der offenkettigen gefärbten Form der radikalischen Zerstörung zugänglich sind. Heißhärtende Dentalmaterialien eignen sich besonders zur Herstellung von Inlays und Onlays.

Dauerhaft reversible photochrome Dentalwerkstoffe auf der Basis organischer photochromer Farbstoffe lassen sich auch mit selbst- bzw. kalthärtenden sowie dual härtbaren Systemen herstellen. Bei einem selbst- bzw. kalthärtenden System wird eine aminhaltige Basenpaste mit einer peroxidhaltigen Katalysatorpaste gemischt. Durch die Reaktion von Amin und Peroxid wird die radikalische Polymerisation initiert. Bevorzugter Katalysator ist Dibenzoylperoxid.

Bei dual härtbaren Systemen enthält die Basenpaste zusätzlich einen Photoinitiator, wie beispielsweise Campferchinon, so daß die Basenpaste entweder allein als lichthärtender oder zusammen mit der Katalysatorpaste als licht- und selbsthärtender Dentalwerkstoff eingesetzt werden kann.

Bei selbst- und dual härtenden Systemen ist die Reversibilität des Farbumschlags von dem Verhältnis von Katalysator zu photochromem Farbstoff abhängig. Bei Verwendung des bevorzugten Katalysators Dibenzoylperoxid und einer üblichen Katalysatorkonzentration von beispielsweise etwa 0,75 Gew.-% Dibenzoylperoxid (50 %ig) ist eine Farbstoffkonzentration von 0.01 bis 0.1 Gew.-% bevorzugt. Auch bei selbst- und dual härtenden Systemen wird eine reversible Photochromie nur dann erzielt, wenn die Härtung des Dentalmaterials im ungefärbten Zustand erfolgt.

Bei den bevorzugten photochromen Farbstoffen beträgt die Entfärbungszeit bei reversibel photochromen Dentalmaterialien sowohl im Fall der heiß- als auch der selbst- und dual härtenden Systeme etwa 2 Stunden, vorzugsweise etwa 1 Stunde. Da jedoch die Bearbeitung des Dentalwerkstoffs bzw. die Entfernung überschüssigen Dentalwerkstoffs gewöhnlich im Licht einer OP-Leuchte (Wellenlängenbereich etwa 400 - 700 nm) erfolgt, findet während der Bearbeitung normalerweise keine Entfärbung statt, so daß auch photochrome Materialien mit einer deutlich geringeren Entfärbungszeit erfindungsgemäß geeignet sind.

Erfindungsgemäß geeignete photochrome Gläser werden beispielsweise in der US-A-4 891 336, US-A-4 979 976 und der EP 0 422 514 A1 offenbart. Hierbei handelt es sich um photochrome Gläser auf der Basis von Metallhalogeniden.

Besonders geeignet sind Silicium-Aluminium-Borat-Gläser deren photochromer Effekt auf dem Zusammenspiel von Silber, Chlor, Brom und Kupfer jeweils in mehreren unterschiedlichen Oxidationsstufen basiert. Besonders bevorzugte Gläser werden in der DE 30 36 103 C 2, US-A-3 208 860 und in der US-A-4 046 781 beschrieben. Ganz besonders bevorzugt sind Gläser mit der Zusammensetzung:

| Bestandteil | Gew.-% |
|---|---|
| $SiO_2$ | 48,0 - 60,0 |
| $Al_2O_3$ | 5,0 - 12,0 |
| $B_2O_3$ | 16,0 - 25,0 |
| $Li_2O$ | 1,6 - 3,5 |
| $Na_2O$ | 3,0 - 7,0 |
| $K_2O$ | 5,0 - 10,0 |
| $TiO_2$ | 1,8 - 2,2 |
| $ZrO_2$ | 4,0 - 6,0 |
| Ag | 0,15 - 0,5 |
| CuO | 0,005 - 0,02 |
| Cl | 0,15 - 0,25 |
| Br | 0,05 - 0,15 |

Solche Gläser werden beispielsweise von der Deutschen Spezialglas AG unter der Bezeichnung "Photosolar Supergrey D-1426" vertrieben.

Dentalwerkstoffe auf der Basis von metallhalogenidhaltigen Gläsern, Keramiken oder Glaskeramiken verfärben sich bei der Bestrahlung mit Licht dunkel. Sie reagieren dabei im allgemeinen auf das ganze Spektrum des sichtbaren Lichts empfindlich, ohne ein besonders ausgeprägtes Absorbtionsmaximum zu zeigen. Im allgemeinen ist eine Bestrahlungszeit von 20 bis 40 Sekunden zur deutlichen Sichtbarmachung des Dentalwerkstoffs ausreichend.

Die Färbung der photochromen Gläser bei der Belichtung beruht auf der Reduktion von ionischem Silber (Zustand A) zu elementarem Silber (Zustand B), das unter Lichtausschluß bei gleichzeitiger Entfärbung wieder oxidiert wird. Die vollständige Entfärbung erfolgt vorzugsweise innerhalb von 0,5 bis 1,5 Stunden.

Dentalwerkstoffe auf der Basis von photochromen Gläsern, Keramiken oder Glaskeramiken zeigen in der Regel eine dauerhafte Reversibilität der Photochromie, und sie verlieren ihre Fähigkeit zur Photochromie auch dann nicht, wenn der mit ihnen ausgestattete Dentalwerkstoff, wie zum Beispiel ein Füllungsmaterial, über einen längeren Zeitraum den chemischen und physikalischen Einflüssen ausgesetzt ist, die auf die natürlichen Zähne einwirken. Aus ästhetischen Gründen eignen sich diese Materialien daher bevorzugt für den Einsatz im Seitenzahnbereich oder als Unterfüllungsmaterialien.

Photochrome Gläser, Keramiken und/oder Glaskeramiken werden bevorzugt als Füllstoffe eingesetzt, vorzugsweise in einer Konzentration von 10 bis 90 Gew.-%, besonders bevorzugt 30 bis 60 Gew.-% bezogen auf die Gesamtmasse des Dentalwerkstoffs. Für die Anwendung in Dentalwerkstoffen sind Gläser in Pulverform mit einer mittleren Korngröße von 0,7 bis 20 µm, insbesondere 0,7 bis 5 µm sowie Gläser mit einem Brechungsindex von 1,50 bis 1,58 bevorzugt. Die Wahl des Polymerisationskatalysators ist ohne Einfluß auf die Photochromie der Gläser, Keramiken und Glaskeramiken.

Die erfindungsgemäßen photochromen Materialien sind mit den unterschiedlichsten Dentalwerkstoffen verträglich und erweisen sich besonders bei der Einarbeitung in farblose oder zahnfarbene Dentalwerkstoffe als vorteilhaft, da sie im wesentlichen zu keiner sichtbaren Verfärbung des Materials führen.

Dentalwerkstoffe im Sinne der Erfindung sind insbesondere Komposit-Füllungsmaterialien, Befestigungskunststoffe für Inlays, Onlays, Kronen und Brücken sowie Ausblockmaterialien.

Bevorzugt sind Dentalwerkstoffe auf Basis eines polymerisierbaren, ethylenisch ungesättigten Monomeren als Bindemittel, eines Katalysators für die Heiß-, Kalt- und/oder Photopolymerisation und 20 bis 90 Gew.-% eines anorganischen Füllstoffs.

Als polymerisierbare organische Bindemittel eignen sich alle für einen Dentalwerkstoff brauchbaren Bindemittel, insbesondere monofuktionelle oder polyfunktionelle Methacrylate, die allein oder in Mischungen eingesetzt werden können. Als Beispiele für diese Verbindungen kommen Methylmethacrylat, Isobutylmethacrylat, Cyclohexylmethacrylat, Tetraethylenglycoldimethacrylat, Triethylenglycoldimethacrylat, Diethylenglycoldimethyacrylat, Ethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, 2,2-Bis-4-(3-methacryloxy-2-hydroxy-propoxy)-phenylpropan (Bis-GMA) sowie die Reaktionsprodukte aus Isocyanaten, insbesondere Di- und/oder Triisocyanaten und OH-gruppenhaltigen Methacrylaten in Frage. Beispiele dafür sind die Umsetzungsprodukte von 1 Mol Hexamethylendiisocyanat mit 2 Mol 2-Hydroxyethylenmethacrylat, von 1 Mol Tri-(6-isocyanatohexyl)biuret mit 3 Mol 2-Hydroxyethylmethacrylat und (6-isocyanatohexyl)biuret mit 3 Mol 2-Hydroxyethylmethacrylat und von 1 Mol 2,2,4-Trimethylhexamethylendiisocyanat mit 2 Mol 2-Hydroxyethylmethacrylat.

Als Katalysatoren für die heiß-härtenden Systeme sind Peroxide, insbesondere Dibenzoylperoxid, Dilauroylperoxid, tert.-Butylperoctat und tert.-Butylperbenzoat bevorzugt. Darüber hinaus sind auch 2,2'-Azoisobuttersäurenitril (AIBN), Benzpinakol und 2,2'-Dialkylbenzpinakole geeignet.

Als Katalysatoren für die Kaltpolymerisation werden Radikale liefernde Systeme, zum Beispiel Benzoyl- bzw. Lauroylperoxid zusammen mit Aminen wie N,N-Dimethyl-p-toluidin, N,N-Dihydroxyethyl-p-toluidin oder anderen strukturverwandten Aminen eingesetzt.

Als Initiatoren für die Photopolymerisation können zum Beispiel Benzophenon und seine Derivate sowie Benzoin und seine Derivate verwendet werden. Weitere bevorzugte Photoinitiatoren sind die α-Diketone wie 9,10-Phenanthrenchinon, Diacetyl, Furil, Anisil, 4,4'-Dichlorbenzil und 4,4'-Dialkoxybenzil. Besonders bevorzugt wird Campherchinon verwendet.

Als Katalysatoren für dual härtbare Systeme eignen sich Kombinationen aus Kalt- und Photokatalysatoren. Bevorzugt ist die Verwendung von Campherchinon und Dibenzoylperoxid in Kombination mit den oben genannten Aminen.

Als anorganische Füllstoffe werden z.B. Quarz -,Glaskeramik - oder Glaspulver, die Oxide von Aluminium oder Silicium, Bariumsilikatgläser und Li/Al Silikatgläser, Bariumgläser, feinstteilige Kieselsäuren, insbesondere pyrogene oder gefällte Kieselsäuren verwendet.

Geeignete Füllstoffe werden beispielsweise in der DE-OS 40 29 230 offenbart. Füllstoffe des Typs (A) sind in der DE-PS 32 47 800 beschrieben.

Die erfindungsgemäßen photochromen Gläser werden vorzugsweise als Komponente (B) verwendet, entweder allein, oder in Kombination mit einem Bariumsilikatglas, daß die erforderlichen Parameter aufweist.
Im folgenden wird die Erfindung anhand von Beispielen näher erläutert.

**Beispiel 1**

**Photochromer, lichthärtender zahnfarbener Kompositzement**

Durch Mischen der Komponenten wird eine Basispaste mit folgender Zusammensetzung hergestellt (analog zu DE 40 29 230 A1) :

| Komponente | Gew.-% |
|---|---|
| Ba-Al-Silikatglas, silanisiert | 40,0 |
| Ytterbiumtrifluorid | 25,0 |
| sphäroides Mischoxid, silanisiert* | 10,0 |
| Bisphenol-A-glycidyldimethacrylat (Bis-GMA) | 12,28 |
| Triethylenglycoldimethacrylat (TEGDMA) | 6,22 |
| Urethandimethacrylat (UDMA) ** | 6,22 |
| Campherchinon | 0,07 |
| Cyanoethylmethylanilin | 0,07 |
| N,N-Diethyl-3,5-di-*tert.*-butylanilin | 0,1 |
| 3,5-Di-*tert.*-butyl-4-hydroxytoluol (BHT) | 0,03 |
| HD 579 | 0,01 |

* Füllstoff A gemäß DE 40 29 230 A1

** Reaktionsprodukt aus 1 Mol Trimethylhexamethylendiisocyanat und 2 Mol Hydroxyäthylmethacrylat

Hierzu werden in eine Monomermischung aus Bis-GMA, TEGDMA, UDMA, Campherchinon und Cyanoethylmethylanilin, N,N-Diethyl-3,5-di-*tert.*-butylanilin, BHT und als Füllstoffe silanisiertes Ba-Al-Silikatglas, Ytterbiumtrifluorid und sphäroides Mischoxid eingearbeitet. Zusätzlich werden 0,01 Gew.-% des photochromen Farbstoffs HD 579 (Tabelle 1) zugegeben.
Weiterhin wird eine Katalysator-Paste mit der folgenden Zusammensetzung hergestellt (analog zu DE 40 29 230 A1):

| Komponente | Gew.-% |
|---|---|
| Ba-Al-Silikatglas, silanisiert | 40,0 |
| Ytterbiumtrifluorid | 25,0 |
| sphäroides Mischoxid, silanisiert | 10,0 |
| Bis-GMA | 12,22 |
| TEGDMA | 6,0 |
| UDMA | 6,0 |
| Benzoylperoxid (50 %-ig) | 0,75 |
| BHT | 0,03 |

Hierzu werden in eine Monomermischung aus Bis-GMA, TEGDMA, UDMA, BHT und 50-%iges Benzoylperoxid, als Füllstoffe silanisiertes Ba-Al-Silikatglas, Ytterbiumtrifluorid und sphäroides Mischoxid eingearbeitet.
Die Pasten werden im Verhältnis 1 : 1 gemischt und mit dem zu zementierenden Teil (Krone, Brücke, Veneer, Inlay, Onlay) eingesetzt. Der aus dem Zementierungsspalt austretende Zement wird für 1 bis 3 Sekunden mit einer handelsüblichen Polymerisationslampe (Heliolux® GTE, Firma Vivadent) bei einer Wellenlänge von 400 bis 500 nm bestrahlt. Der Zement färbt sich schlagartig intensiv rot, bleibt aber dünnviskos. In dieser Phase können die gröbsten Überschüsse leicht entfernt werden.
Nach einer weiteren Bestrahlung von etwa 20 Sekunden polymerisiert der Zement zu einer harten Masse, die weiterhin rot gefärbt ist. Die auspolymerisierten Überschüsse bleiben gut sichtbar und können exakt entfernt werden.
Durch eine zusätzliche Bestrahlung von 40 bis 60 Sekunden wird der Zement fast vollständig entfärbt. Die

verbleibende schwache Rosafärbung verschwindet nach Lagerung ohne Bestrahlung innerhalb von 24 Stunden. Die ausgehärtete Masse zeigt ein zahnfarbenes Aussehen. Die Entfärbung ist irreversibel, da der verwendete Farbstoff durch die radikalische Polymerisation zerstört wird.

**Beispiele 2 bis 10**

Gemäß Beispiel 1 wird eine Basispaste hergestellt, wobei jedoch als photochrome Materialien die in Tabelle 1 genannten Farbstoffe verwendet werden. Die Basispasten werden mit der Katalysatorpaste gemäß Beispiel 1 gemischt und wie in Beispiel 1 beschrieben bestrahlt. Die Dentalwerkstoffe zeigen in Abhängigkeit von dem gewählten Farbstoff unterschiedliche Färbungen (Tabelle 1) und Farbintensitäten. Die Entfärbung ist in allen Fällen irreversibel.

**Beispiel 11**

**Photochromes Füllungsmaterial**

Durch Mischen der Komponenten wird eine Masse mit der folgenden Zusammensetzung hergestellt:

| Komponente | Gew.-% |
|---|---|
| Photosolar supergrey D-1426* | 15,0 |
| Ba-Al-Silikatglas, silanisiert | 35,0 |
| Ytterbiumtrifluorid | 15,0 |
| sphäroides Mischoxid, silanisiert | 15,0 |
| Aerosil OX-50,** silanisiert | 1,0 |
| Bis-GMA | 8,0 |
| TEGDMA | 3,8 |
| UDMA | 7,02 |
| Monomethylhydrochinon (MeHQ) | 0,02 |
| Campherchinon | 0,06 |
| Cyanoethylmethylanilin | 0,1 |

* Photochromes Glas der Deutschen Spezial Glas AG, das Glas wird auf eine mittlere Korngröße von 0,7 bis 5 µm gemahlen

** Aerosil OX-50 (Degussa AG)

Die Masse wird wie ein gewöhnliches Füllungsmaterial in eine Kavität eingebracht und für 40 bis 60 Sekunden mit einer Polymerisationslampe (Heliolux GTE, Firma Vivadent) mit einer Wellenlänge von 400 bis 500 nm bestrahlt. Bei der Bestrahlung der Paste härtet das Komposit aus und verfärbt sich zugleich grau. Innerhalb einer etwa 1,5-stündigen Lagerung unter Lichtausschluß entfärbt sich der Prüfkörper wieder und zeigt ein zahnfarbenes Aussehen. Bei erneuter Bestrahlung (ca. 10 Sekunden) des Prüfkörpers verfärbt sich dieser abermals grau, nach Lagerung unter Lichtausschluß findet wieder eine Entfärbung statt. Dieser Prozeß läßt sich beliebig oft wiederholen.

**Tabelle 1**

**Derivate der Verbindung I**

| Beispiel | Farbstoff | X | $R^1$ | $R^2$ | $R^3$ | $R^4$ | $R^5$ | $R^6$ | $R^7$ | Farbe |
|---|---|---|---|---|---|---|---|---|---|---|
| 1 | HD 579 | N | H | H | H | $CH_3$ | - | $COOCH_3$ | $COOCH_3$ | rot |
| 2 | HD 578 | N | H | H | H | H | - | $COOCH_3$ | $COOCH_3$ | rot |
| 3 | HD 580 | N | H | H | $COOCH_3$ | H | - | $COOCH_3$ | $COOCH_3$ | rot |
| 4 | HD 581 | N | H | H | CN | H | - | $COOCH_3$ | $COOCH_3$ | rot |
| 5 | HD 582 | N | $CH_3$ | H | H | $CH_3$ | - | $COOCH_3$ | $COOCH_3$ | grün |
| 6 | HD 604 | N | H | $-(CH_2=CH_2)_2-$ | | H | - | $COCH_3$ | $COCH_3$ | blauviolett |
| 7 | HD 606 | N | H | H | H | H | - | $COCH_3$ | $COOCH_3$ | rot |
| 8 | HD 607 | C-$R^5$ | H | $CH_2=C-(CH_3)-COO-$ | H | H | H | $COOCH_3$ | $COOCH_3$ | grün |
| 9 | HD 608 | C-$R^5$ | H | H | H | H | $CH_2=C(CH_3)-COO-$ | $COOCH_3$ | $COOCH_3$ | grün |
| 10 | HD 609 | C-$R^5$ | H | H | $CH_2=C(CH_3)-COO-$ | H | H | $COOCH_3$ | $COOCH_3$ | grün |

**Patentansprüche**

1. Dentalwerkstoff, **dadurch gekennzeichnet, daß** er ein photochromes Material enthält.

2. Dentalwerkstoff gemäß Anspruch 1, **dadurch gekennzeichnet, daß** er als photochromes Material einen photochromen Farbstoff, ein photochromes Glas, eine photochrome Keramik und/oder eine photochrome Glaskeramik enthält.

3. Dentalwerkstoff gemäß Anspruch 2, **dadurch gekennzeichnet, daß** er als photochromen Farbstoff mindestens ein photochromes Spiro[1,8a-indolizin]-Derivat enthält.

4. Dentalwerkstoff gemäß Anspruch 3, **dadurch gekennzeichnet, daß** er mindestens ein Spiro[1,8a-dihydroindolizin]- und/oder Spiro[1,8a-tetrahydroindolizin]-Derivat enthält.

5. Dentalwerkstoff gemäß Anspruch 4, **dadurch gekennzeichnet, daß** er mindestens ein Spiro[fluoren-9,1'[1,8a] dihydroindolizin]-Derivat enthält.

6. Dentalwerkstoff gemäß Anspruch 5, **dadurch gekennzeichnet, daß** er mindestens ein Spiro[fluoren-9,1'[1,8a] dihydroindolizin]-Derivat gemäß der Formel

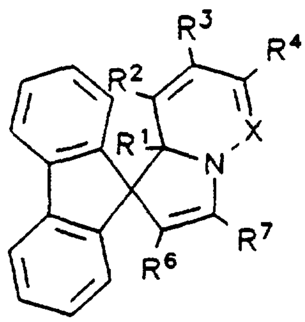

enthält, in der

X C-$R^5$ oder N;
$R^1$ H oder $CH_3$;
$R^2$ H, $CH_2$=C($CH_3$)-COO- oder zusammen mit $R^3$ einen ankondensierten Benzolring (-($CH_2$=$CH_2$-$)_2$);
$R^3$ H, $CH_3$, $COOCH_3$, CN, $CH_2$=C($CH_3$)-COO- oder zusammen mit $R^2$ einen ankondensierten Benzolring (-($CH_2$=$CH_2$-$)_2$);
$R^4$ H oder $CH_3$;
$R^5$ H oder $CH_2$=C($CH_3$)-COO-;
$R^6$ $COOCH_3$ oder $COCH_3$ und
$R^7$ $COOCH_3$ oder $COCH_3$

bedeuten.

7. Dentalwerkstoff gemäß Anspruch 2, **dadurch gekennzeichnet, daß** er ein photochromes Glas enthält, das die Komponenten

| | |
|---|---|
| $SiO_2$ | 48,0 - 60,0 Gew.-% |
| $Al_2O_3$ | 5,0 - 12,0 Gew.-% |
| $B_2O_3$ | 16,0 - 25,0 Gew.-% |
| $Li_2O$ | 1,6 - 3,5 Gew.-% |
| $Na_2O$ | 3,0 - 7,0 Gew.-% |

(fortgesetzt)

| | |
|---|---|
| $K_2O$ | 5,0 - 10,0 Gew.-% |
| $TiO_2$ | 1,8 - 2,2 Gew.-% |
| $ZrO_2$ | 4,0 - 6,0 Gew.-% |
| Ag | 0,15 - 0,5 Gew.-% |
| CuO | 0,005 - 0,02 Gew.-% |
| Cl | 0,15 - 0,25 Gew.-% |
| Br | 0,05 - 0,15 Gew.-% |

umfaßt.

**8.** Dentalwerkstoff gemäß einem der Ansprüche 1 bis 7, **dadurch gekennzeichnet, daß** er zusätzlich ein ethylenisch ungesättigtes Monomer, einen Katalysator für die Heiß-, Kalt- und/oder Photopolymerisation und 20 bis 90 Gew.-% eines anorganischen Füllstoffs enthält.

**9.** Verwendung eines photochromen Materials zur Herstellung eines Dentalwerkstoffs.

## Claims

**1.** Dental material, **characterized in that** it contains a photochromic material.

**2.** Dental material according to Claim 1, **characterized in that** it contains, as photochromic material, a photochromic dye, a photochromic glass, a photochromic ceramic and/or a photochromic glass ceramic.

**3.** Dental material according to Claim 2, **characterized in that** it contains, as photochromic dye, at least one photochromic spiro[1,8a-indolizine] derivative.

**4.** Dental material according to Claim 3, **characterized in that** it contains at least one spiro[1,8a-dihydroindolizine] and/or spiro[1,8a-tetrahydroindolizine] derivative.

**5.** Dental material according to Claim 4, **characterized in that** it contains at least one spiro[fluorene-9,1'[1,8a]-dihydroindolizine] derivative.

**6.** Dental material according to Claim 5, **characterized in that** it contains at least one spiro[fluorene-9,1'[1,8a]-dihydroindolizine] derivative according to the formula

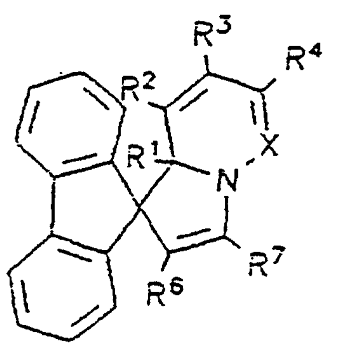

in which

X is C-$R^5$ or N;

$R^1$ is H or $CH_3$;

$R^2$ is H, $CH_2=C(CH_3)$-COO- or, together with $R^3$, a fused benzene ring ($-(CH_2=CH_2-)_2$);

$R^3$ is H, $CH_3$, $COOCH_3$, CN, $CH_2=C(CH_3)$-COO- or, together with $R^2$, a fused benzene ring ($-(CH_2=CH_2-)_2$) ;

$R^4$ is H or $CH_3$;

$R^5$ is H or $CH_2=C(CH_3)$-COO-;

$R^6$ is $COOCH_3$ or $COCH_3$ and

$R^7$ is $COOCH_3$ or $COCH_3$.

**7.** Dental material according to Claim 2, **characterized in that** it contains a photochromic glass which comprises the components

| | |
|---|---|
| $SiO_2$ | 48.0-60.0% by weight |
| $Al_2O_3$ | 5.0-12.0% by weight |
| $B_2O_3$ | 16.0-25.0% by weight |
| $Li_2O$ | 1.6-3.5% by weight |
| $Na_2O$ | 3.0-7.0% by weight |
| $K_2O$ | 5.0-10.0% by weight |
| $TiO_2$ | 1.8-2.2% by weight |
| $ZrO_2$ | 4.0-6.0% by weight |
| Ag | 0.15-0.5% by weight |
| CuO | 0.005-0.02% by weight |
| Cl | 0.15-0.25% by weight |
| Br | 0.05-0.15% by weight |

**8.** Dental material according to one of Claims 1 to 7, **characterized in that** it additionally contains an ethylenically unsaturated monomer, a catalyst for the hot, cold and/or photopolymerization and 20 to 90% by weight of an inorganic filler.

**9.** Use of a photochromic material for the production of a dental material.

## Revendications

**1.** Composition dentaire, **caractérisée en ce qu'**elle contient un matériau photochromique.

**2.** Composition dentaire selon la revendication 1, **caractérisée en ce qu'**elle contient comme matériau photochromique, un colorant photochromique, un verre photochromique, une céramique photochromique et/ou une vitrocéramique photochromique.

**3.** Composition dentaire selon la revendication 2, **caractérisée en ce qu'**elle contient comme colorant photochromique au moins un dérivé photochromique de spiro[1,8a-indolizine].

**4.** Composition dentaire selon la revendication 3, **caractérisée en ce qu'**elle contient au moins un dérivé de spiro [1,8a-dihydroindolizine], et/ou spiro[1,8a-tétrahydroindolizine].

**5.** Composition dentaire selon la revendication 4, **caractérisée en ce qu'**elle contient au moins un dérivé de spiro [fluorène-9,1'[1,8a]-dihydroindolizine].

**6.** Composition dentaire selon la revendication 5, **caractérisée en ce qu'**elle contient au moins un dérivé de spiro [fluorène-9,1'[1,8a]-dihydroindolizine] de formule :

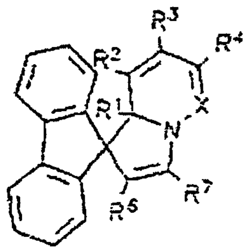

dans laquelle

X désigne C-$R^5$ ou N ;
$R^1$ désigne H ou $CH_3$;
$R^2$ désigne H, $CH_2$ = C($CH_3$)-COO- ou associé à $R^3$ un cycle de benzène condensé (-($CH_2$ = ($CH_{2-}$)-$_2$);
$R^3$ désigne H, $CH_3$, $COOCH_3$, CN, $CH_2$ = C($CH_3$)-COO- ou associé à $R^2$ un cycle de benzène condensé (-($CH_2$ = ($CH_{2-}$)-$_2$);
$R^4$ désigne H ou $CH_3$;
$R^5$ désigne H ou $CH_2$ = C($CH_3$)-COO- ;
$R^6$ désigne $COOCH_3$ ou $COCH_3$ ; et
$R^7$ désigne $COOCH_3$ ou $COCH_3$.

**7.** Composition dentaire selon la revendication 2, **caractérisée en ce qu'**elle contient un verre photochromique, qui comprend les composants :

| | |
|---|---|
| $SiO_2$ | 48,0 à 60,0 % en poids, |
| $Al_2O_3$ | 5,0 à 12,0 % en poids, |
| $B_2O_3$ | 16,0 à 25,0 % en poids, |
| $Li_2O$ | 1,6 à 3,5 % en poids, |
| $Na_2O$ | 3,0 à 7,0 % en poids, |
| $K_2O$ | 5,0 à 10,0 % en poids, |
| $TiO_2$ | 1,8 à 2,2 % en poids, |
| $ZrO_2$ | 4,0 à 6,0 % en poids, |
| Ag | 0,15 à 0,5 % en poids, |
| CuO | 0,005 à 0,02 % en poids, |
| Cl | 0,15 à 0,25 % en poids, |
| Br | 0,05 à 0,15 % en poids. |

**8.** Composition dentaire selon l'une quelconque des revendications 1 à 7, **caractérisée en ce qu'**elle contient en plus un monomère éthyléniquement insaturé, un catalyseur pour une polymérisation à froid, à chaud et / ou une photopolymérisation et 20 à 90 % en poids d'une charge minérale.

**9.** Utilisation d'un matériau photochromique pour la fabrication d'une composition dentaire.